# EUROPEAN PATENT APPLICATION

(11) **EP 2 705 789 A1**
(43) Date of publication of application: **12.03.2014**
(21) Application number: 12183682.9
(22) Date of filing: 10.09.2012
(51) Int. Cl.: A61B 5/0488, A61B 5/0402, A61B 5/0476

(54) **Sensor, monitoring system and method for physiological measurement**

(71) Applicant: GENERAL ELECTRIC COMPANY, Schenectady, NY 12345 (US)
(72) Inventor: Urtti, Tommi, 00710 Helsinki (FI)
(74) Representative: Kanerva, Arto

(57) **Abstract**

A sensor for physiological measurement is disclosed herein. The sensor includes at least one detector (21) for acquiring a signal indicative of vital sign of a subject. The sensor also includes at least one state indicator (19) for indicating based on the signal acquired by the at least one detector one of at least two different ranges, which ranges are a first range and a second range, the first range being for one of desired vital sign signals and minor alarming vital sign signals, and the second range being for vital sign signals outside the first range. A corresponding method and monitoring system is also provided.

## Description

### BACKGROUND OF THE INVENTION

This disclosure relates generally to a sensor, monitoring system and method for physiological measurement.

When monitoring the vital signs or other physiological parameters of hospitalized patients, sensors are attached on patients' skin or catheters are inserted either into natural openings of the body or catheters are pierced through the skin. The common practice is to connect these sensors with electrically or optically conductive cables to measurement instruments. The instrument may reside either on bedside, e.g. multi-parameter patient monitors in an operating room (OR) or in an intensive care unit (ICU) or it may be a relatively small box carried by the patient (e.g. ECG telemetry).

Infection control has become a big issue in a hospital environment. The term "disposable" as used herein refers to a single-use sensor which is used once and then disposed. Totally disposable sensors would make infection control easier. They would also streamline the care process by eliminating the need for cleaning the sensors. The use of disposable single-patient-use sensors prevents the spreading of infections and cross contamination inside the hospital. This also improves the care process by saving time and money. There are several disposable sensors available on the market such as a depth-of-anesthesia sensor, SpO2, ECG sensors, etc.

Recent technological development has made it possible to build battery operated sensors, which include means for performing the actual measurement, converting the measured signals into digital format, and transmitting wirelessly the measurement data and/or calculated parameters to a host device. Instead of transmitting the data in real time, it is also possible to store the data in local memory and download the data afterwards. These devices are referred to as wireless sensors. Wireless sensors provide obvious benefits for both caregivers and patients. The so called 'cable clutter' has been recognized as one of the biggest issues in the care process of high-acuity patients. There are a lot of cables in the hospitals that also create issues with infections and the usability. By using wireless sensors one can reduce the amount of cables used in a hospital and improve the usability of the different parameters and the total care process. It is not necessary to remove all the cables, but a significant improvement would be achieved by removing a moderate number of leads or wires. This is because the tendency to tangle increases disproportionally with the number of cables. The patients that would benefit most from the wireless sensors are low-acuity patients. Being not physically tied to the patient monitor with lead wires, they are free to move around, for example, visiting the bathroom without assistance. Also in case of a small patient monitor carried by the patient, wireless sensors offer better reliability and are more comfortable for the patient.

The measurements that are monitored for each patient depend on their condition. During the monitoring there are times when the measurement values can go above or below the safe range (alarm limits) that are set by the care personnel. In these cases alarms are fired. The purpose of alarms is to alert the care personnel to a change in patient health. Alarms are currently 1) shown on-screen of a traditional patient monitor, 2) alarm lights (leds on the monitor), 3) alarm sounds (speakers in the monitor) and in some cases with remote monitoring tools like through a central. A typical alarm text that would be displayed on-screen could be something like "Sp02 low" with a background color based on the acuteness of the alarm. (Red - yellow -blue are the colors used by some monitors).

In addition to the alarm limits there is another range in some parameters that can be set by the care personnel. This range for the measurements is called "Target Zone" meaning that the ideal value for the measurement would be within this range.

A major clinical issue with the workflow of current monitors and their alarm systems is that when an alarm is firing, the patient's health is typically declining. In these cases it is important for the care personnel to be able to watch the patient closely while maneuvering the required care, but unfortunately they have to watch now and then the measurement results or alarms on the display of the monitor locating apart from the patient.

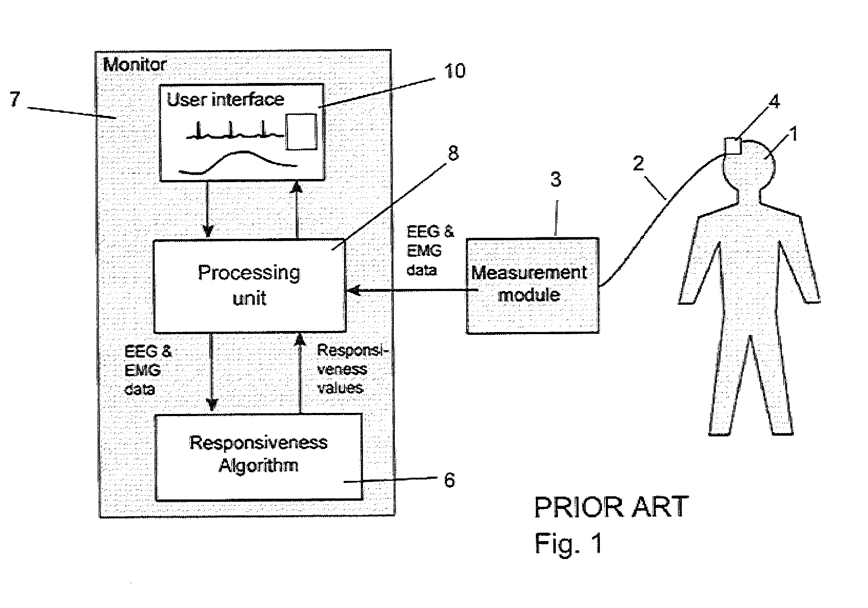

Figure 1 shows as an example a well-known sedation measurement, where a subject 1 is connected along a connection line 2 to a module 3 for receiving a signal indicative of the EEG/EMG received from a sensor 4 attached to a forehead of the subject. The EMG power is calculated by the module from the EEG/EMG signal. A processing unit 8 with a responsiveness algorithm 6 in a monitor 7 is processing the continuous EMG power to a single number called a Responsiveness Index (RI). The processing unit is communicating with a user interface 10 having a display, too. The RI is currently shown on the display of the monitor 7 in a parameter window field (number in range of 0-100) and as a trend. The Responsiveness algorithm is designed to monitor increases in electromyographic (EMG) signal amplitude in intensive care patients. The probability and magnitude of the EMG increases is dependent on the sedation level of the patient.

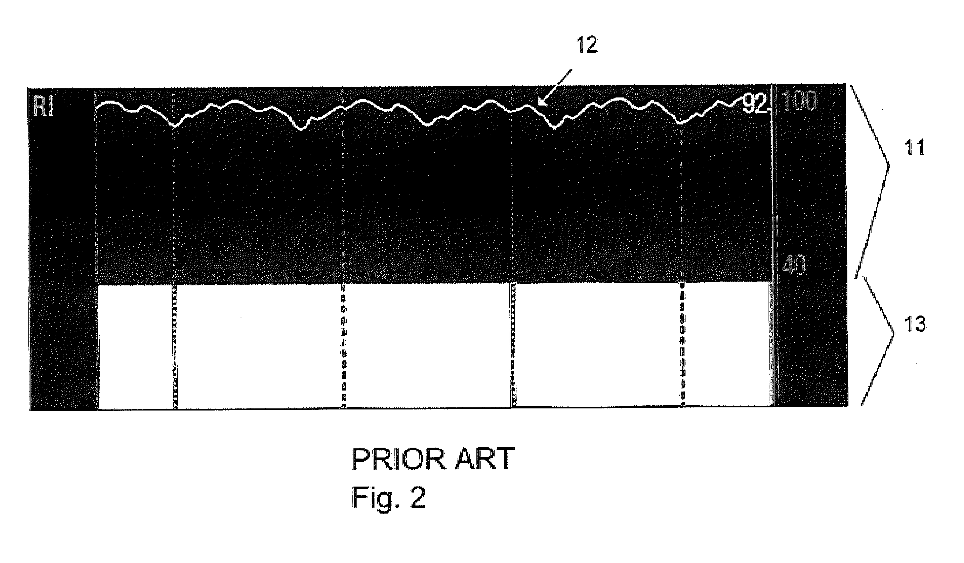

The user needs to monitor the responsiveness index is to know if the patient's sedated level is of the correct depth, which is not too much or too little sedated. On the display of the monitor this is indicated by showing a target zone 11 for the responsive index, which is a bar in the trend area, where the measured RI graph 12 is drawn as shown in Figure 2, If the RI index measurement goes out of the alarm limits bounds (set for the desired depth of sleep) to undesired target zone 13, the patient monitor alarms the care personnel with alarm sounds, alarm light on the patient monitor and on-screen messages.

### BRIEF DESCRIPTION OF THE INVENTION

The above-mentioned shortcomings, disadvantages and problems are addressed herein which will be understood by reading and understanding the following specification.

In an embodiment, a sensor for physiological measurement includes at least one detector for acquiring a signal indicative of vital sign of a subject. The sensor for physiological measurement also includes at least one state indicator for indicating based on the signal acquired by the at least one detector one of at least two different ranges, which ranges are a first range and a second range, the first range being for one of desired vital sign signals and minor alarming vital sign signals, and the second range being for vital sign signals outside the first range.

In another embodiment, a monitoring system for physiological measurement includes a sensor having at least one detector for acquiring a signal indicative of vital sign of a subject. The monitoring system for physiological measurement also includes a monitor unit having processing unit with a measurement algorithm to calculate the signal received from the at least one detector and a user interface for controlling the processing unit, and a first signal connection for transmitting the signal received by the at least one detector to the processing unit. The sensor also comprising at least one state indicator for indicating one of at least two different ranges, which ranges are a first range and a second range, the first range being for one of desired vital sign signals and minor alarming vital sign signals, and the second range being for vital sign signals outside the first range. The monitoring system also comprising a second signal connection for guiding the state indicator based on calculation made by the processing unit to indicate the range of the vital sign signal.

In still another embodiment a method for physiological measurement includes acquiring by means of a sensor a signal indicative of vital sign of a subject and providing the signal to a processing unit provided with a measurement algorithm. The method for physiological measurement also includes calculating by means of the measurement algorithm a vital sign signal value based on the signal and determining in the processing unit a range within which the vital sign signal value belongs to, provided that there are at least two different predetermined ranges, which are a first range and a second range, the first range being for one of desired vital sign signals and minor alarming vital sign signals, and the second range being for vital sign signals outside the first range. The method for physiological measurement also includes comparing in the processing unit the determined range for the vital sign signal value to the at least two different predetermined ranges that have been set for the subject and providing based on the comparing a signal indicating the predetermined range covering the vital sign signal value to a sensor controller. The method for physiological measurement further includes activating by means of the sensor controller a state indicator of the sensor to indicate the appropriate range for the vital sign signal value.

Various other features, objects, and advantages of the invention will be made apparent to those skilled in the art from the accompanying drawings and detailed description thereof.

### BRIEF DESCRIPTION OF THE DRAWINGS

FIG. 1 is a schematic view of prior art embodiment of sedation measurement;

FIG. 2 is a prior art embodiment of a display of monitor;

FIG. 3 is a schematic view of a sensor for physiological measurement in accordance with an embodiment;

FIG. 4 is a schematic view of a monitoring system for physiological measurement in accordance with an embodiment; and

FIG. 5 is a block diagram illustrating a method for physiological measurement in accordance with an embodiment.

### DETAILED DESCRIPTION OF THE INVENTION

Specific embodiments are explained in the following detailed description making a reference to accompanying drawings. These detailed embodiments can naturally be modified and should not limit the scope of the invention as set forth in the claims.

The embodiments relate to a monitor system, sensor and method for physiological measurement allowing clinicians to keep their eyes on a subject. A state indicator for indicating at least one of two different ranges of a vital sign signal, which are a first range and a second range, is indicated, such as displayed or shown, in the direction where the clinician will naturally be looking when caring for the subject. The first range may be for desired vital sign signals and/or minor alarming vital sign signals. The second range may be for vital sign signals outside the first range. The range indication can be done instead/or in addition to displaying it in the monitor.

The range herewith can be understood as the values that form a group that implies a state. Thus each range involved may include a predetermined group of vital sign signals from minimum to maximum. For instance for a measurement (min. 0, max. 100) that has a low alarm set at 20 and high alarm set at 70, there would be three ranges. Consequently values 20-70 thus including a group of vital sign signals would form the first range where no alarms are indicated, this can be understood as the desired range. Values 71-100 including again a group of vital sign signals would form the second range that activates the alarm that the measurement is too high. Values 0-19 would form the third range that activates an alarm that the measurement is too low. Ranges may be formed by alarm limits including several limits for different severities, target zone limits and the absolute maximum/minimum that the said measurement can have.

The vital sign signal typically can be an electrophysiological or bioelectric signal obtained from the subject, for instance an electromyographic (EMG), electroencephalographic (EEG) or electrocardiogram (ECG) signal. Bioelectric signal is generated by nerve cells and/or muscle cells. Also the vital sign signal can be biomechanical signal such as a blood pressure or bio-optical signal. Biomechanical signal originate from some mechanical function of the biologic system, such as pressure, tension, flow and volume change. Typically the signal frequency of vital sign signals explained hereinbefore is more than 0,1 Hz.

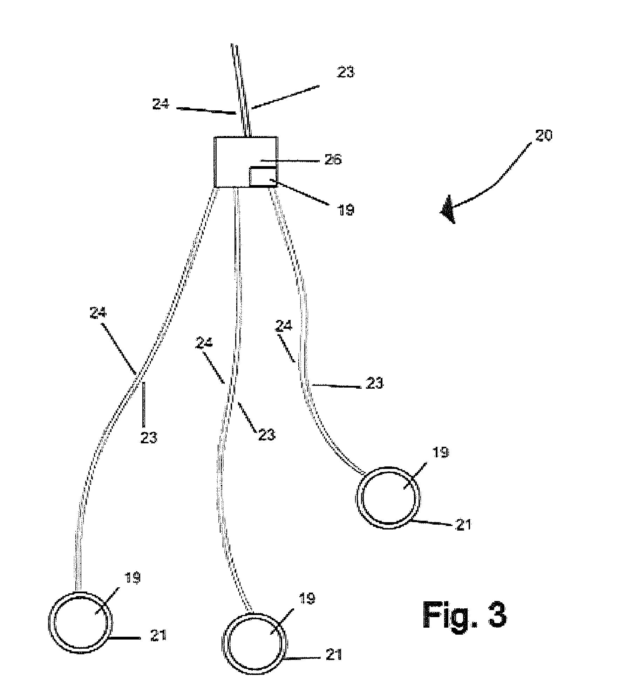

In Figure 3 there is shown a sensor 20 in this specific embodiment for sedation measurement on a head of the subject. The sensor comprises three different detectors 21, such as electrodes, but the number of detectors could be lower or higher depending on the solution. The sensor also comprises at least one state indicator 19, such as a visual indicator or audio indicator, to indicate the range of the at least one vital sign of the subject, which in this specific sedation measurement is at least one of electromyographic (EMG) signal or electroencephalographic (EEG) signal. In Figure 3 the number of state indicators is three, but for instance in EEG and EMG measurements the number of indicators can also be much more or only one. The state indicator 19 is advantageously with at least one of the detectors 21, but it may be with other parts of the sensor, too. It may lie on the detector to make it for instance visually detectable.

The sensor may comprise a substrate (not shown in the Figure) for the detector to attach to the subject or the detector is directly without the substrate attachable to the subject. The detector housing or the substrate may be provided with an adhesive to keep it in place, but naturally mechanical solutions or tapes can be used for that purpose, too.

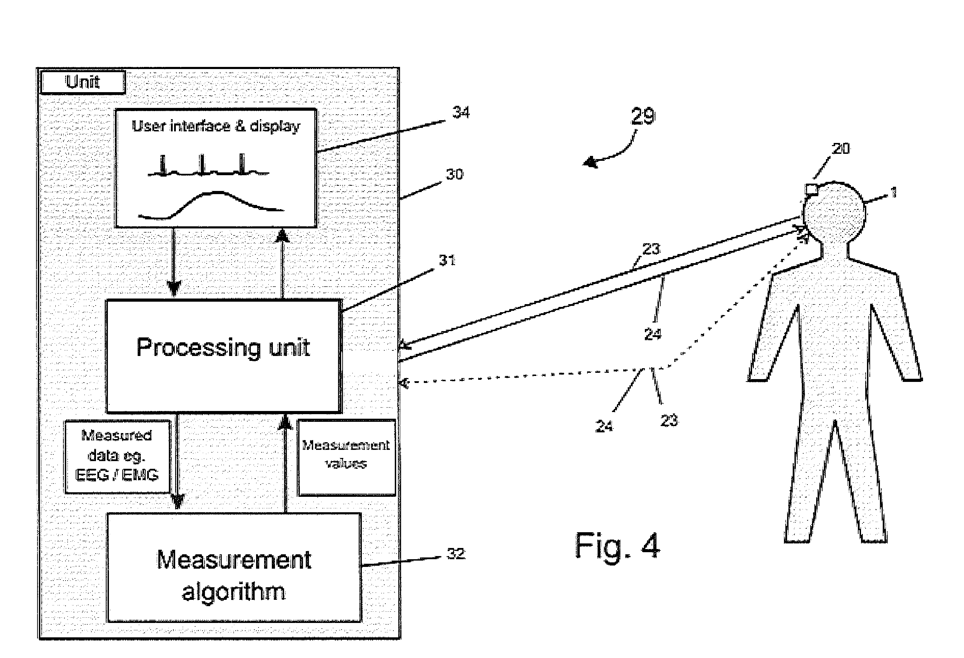

One or more detectors of the sensor with electrical functions are connected along a first signal connection 23 to transmit a signal to a monitor unit 30 shown in Figure 4 for calculating, based on signals provided by the sensor 20 or its detectors 21, the predetermined vital sign. There is a second signal connection 24 between the sensor 20 and the monitor unit 30 for guiding the state indicator 19 to indicate the range of the vital sign calculated by the monitor unit. So the proper range may be indicated on grounds of calculations made by the monitor unit or some processing unit. Both the first and second signal connection can be wireless or wired connections. The sensor may further comprise a sensor controller 26 that activates the state indicator 19 on the detector 21and/ or on the sensor controller as shown in Figure 3, but the sensor controller may locate also in the monitor unit 30.

In accordance with embodiments the sensor can indicate the range of the vital sign signals which may include, if desired, clinical alarms of the subject and target range information especially based on the measured physiological parameters. The sensor, such as a biomedical sensor, featuring at least one of two different ranges can be for instance a lead sensor attached to a skin, a biomechanical sensor attached to an ann or another type of biomedical sensor. The motives for this are 1) improving the observation, such as visibility or audibility, of the range of the vital sign signal and 2) taking the care personnel's' eyes off the monitor and instead on the patient thus alleviating cognitive load and distraction.

As explained hereinbefore there can be at least two different ranges for the at least one vital sign signal, typically three different ranges within which measurement values made by means of the sensor belong to. Naturally there can be more than three different ranges depending on the sensor type or need to show different ranges of the measurement values. At least one of two different ranges, which is a first range, can include at least one of desired vital sign signals, such as allowed vital sign signals, and minor alarming vital sign signals and another of two different ranges, which is a second range, can include vital sign signals outside the first range. The first range, which may include minor alarming signals can be less alarming than the vital sign signals of the second range, which are more alarming, such as medium priority alarm, and showing that something should be made to return to the desired measurement values.

The vital sign signals within the certain range may also belong to two different alarm categories, such as minor alarming and more alarming signals or alternatively without alarming and alarming signals. Apart from the first and second range in some cases a third range is desired for instance to indicate measurement values which are still more alarming, such as high priority alarm, than the second measurement values. In case there is a reason to know whether the measurement values are above or below the first range, there is a need for both the second range and third range. The second range could be indicating measurement values above the first range and the third range indicating measurement values below the first range. It might be advantageous to indicate second and third ranges by giving an alarm signal together with the signal indicating the range or without the signal indicating the range.

A state indicator 19, which is visual, such as a color indicator, can be for example printed electronic ink indicator or a light-emitting diode (LED), on the sensor 20, especially on the detector 21 or on the sensor controller 26.They are two well-known principles and suitable to indicate the range of the vital sign. The detector can show different colors for each range. Also one range could be without color if desired. The first range including desired measurement values or less alarming results can be for example blue, the second range can be yellow for medium priority alarm and red for high priority alarm.

The function of the state indicator can be based on electrochromism, which is a phenomenon displayed by some materials of reversibly changing color when a burst of electric charge is applied. Various types of materials and structures can be used to construct electrochromic devices, depending on the specific applications. The color change is persistent and energy needs only be applied to effect a change of the color. The best-known application of electrochromic materials is electronic paper, or e-paper. E-paper, or electronic ink display, is a display technology designed to mimic the appearance of ordinary ink on paper. Unlike a conventional flat panel display, which uses a backlight to illuminate its pixels, electronic paper reflects light like ordinary paper. It is capable of holding text and images indefinitely without drawing electricity, while allowing the image to be changed later. As used herein, the terms electrochromism and electrochromic are intended to also encompass electrophoresis and electrophoresic materials as alternative phenomena and materials for implementing a bi-stable indicator. Electrophoresis is the motion of charged particles suspended in a liquid in response to an electric field. Positively charged particles move toward the cathode, and negatively charged particles move toward the anode. If these particles are colored, the display shows different colors to the user as the particles move. Thus, electrophoresis may be used to switch pixels on and off and to change color of an indicator or display.

In exemplary embodiments a bi-stable electrochromic state indicator in a biomedical sensor consumes energy only when the color is changed, i.e. when a relatively small voltage is applied. Basically no electric power may be needed to maintain the color change. Hence, the power consumption is very small. Also the information of the state indicator 19 remains visible, even if a battery runs out. Thus, a bistable electrochromic state indicator is ideal for a battery-operated, self-powered biomedical sensor.

The state indicator 19 could be as well the audio indicator in the sensor 20 apart from the monitor unit 30 and would generate sound signals relevant to different ranges and clinically relevant alarms that the monitor does currently. This would be done for example if a standalone measurement device would be created or if the monitor unit would not have sounds. The sounds that a medical device should sound are defined in the standard IEC 60601-1-8.

A monitoring system 29 comprising the monitoring unit 30 and the sensor 20 is shown in Figure 4. The monitoring system may also comprise a measurement module (not shown in Figure) between the monitoring unit and the sensor, in which case the module could take care of part of the functions of the monitor unit. Thus in case the module is included in the monitoring system it is considered part of the monitor unit. The monitoring system further comprises the first signal connection 23 and second signal connection 24, which can be part of the sensor or the monitor unit or they can be separate components. The sensor 20 is communicating along the first signal connection 23 with the monitor unit 30 having a processing unit 31 for making calculations with a measurement algorithm 32 based on signals received from the sensor 20. The second signal connection 24 is for guiding the state indicator 19 based on calculations made by the processing unit 31. As explained hereinbefore the first signal connection 23 and second signal connection 24 can be wired lines, but they can also be wireless, both of which signal connection types are shown in Figure 4 using same reference numbers, but wireless connections are shown with dotted lines and wired lines are shown with solid lines. The monitor may also comprise a user interface 34 including a display. The user interface is controlling the processing unit 31, and the display is showing the data received from the processing unit. When the measurement values cross the threshold values advantageously given by the user to these different ranges as calculated in the processing unit 31 of the monitor unit 30, the signal is transmitted along the second signal connection 24 to a sensor controller 26, if not in the monitoring unit 30, to activate the state indicator 19 on the detector 21. Also the threshold values may be determined in the factory, but typically the user desires to customize these values for different ranges.

The sensor could also comprise the processing unit 31 with the measurement algorithm to make it independent from the separate monitor unit (not shown in Figures). In that case for the processing unit and one or more detectors also a current source, such as battery, is needed. Then there is necessarily no need to communicate with the monitoring unit, but naturally such unit can also exist if desired. The current source in the sensor is also needed in case the sensor is wirelessly communicating with the monitor unit. Also the user interface can be included in the sensor.

This technology could be an important part in the future when physical patient monitors as we know them today are phased out. As explained hereinbefore in this case the algorithms calculation could be done in the sensor instead of passing the biosignal to the monitor and getting back the range of the at least one vital sign information from the monitor.

The embodiment regarding the sensor, such as the sedation sensor, described hereinbefore with more detail is an example, it should not be considered that this same principle could not be applied to different sensors, which typically are attachable to the subject, such as to the skin or cavities of the subject. The same principle could be applied to any measurement that outputs a numeric or other measurement value that is compared to different ranges, one of those ranges may indicate alarm limit(s) or target-range.

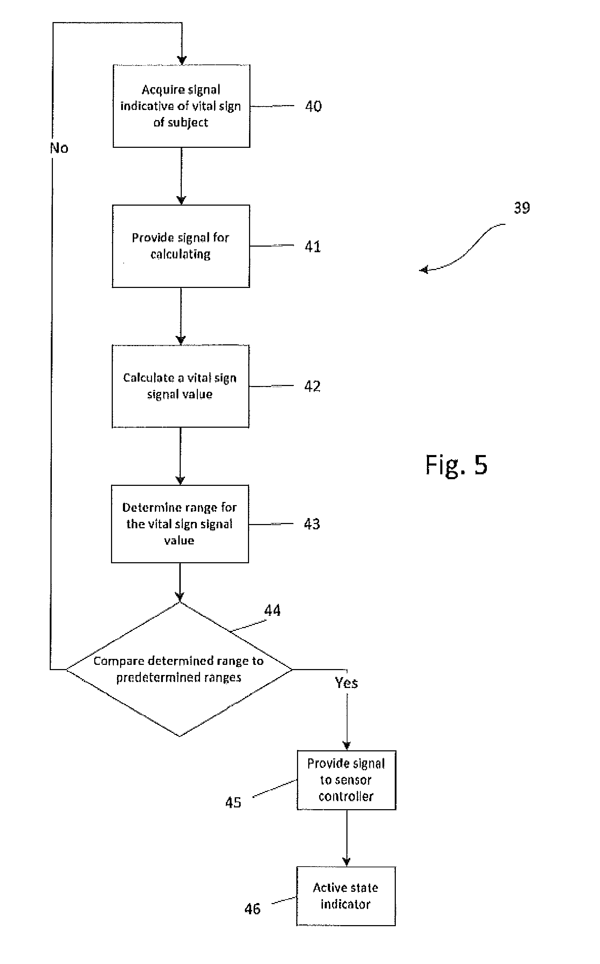

Figure 5 shows an embodiment of a physiological measurement method 39. At step 40 a signal indicative of the vital sign, like EEG or EMG, is acquired by means of the sensor 20 having the detector(s) 21, such as electrode(s). The signal is provided at step 41 for calculating to the processing unit 31 that could be monitoring software running on a patient monitor or an embedded, wearable device. The processing unit could also be a cloud computing solution. By means of the measurement algorithm 32 a vital sign signal value based on the signal is calculated at step 42. The processing unit 31 determines the range within which the calculated vital sign signal value belongs to at step 43. As explained hereinbefore at least two different ranges were predetermined. The outcome value could for instance be 62 on a maximum range of 0 to 100. The processing unit 31 is comparing at step 44 the determined range of the vital sign signal value to predetermined ranges that have been set for the subject. According to step 45 the signal indicating the predetermined range within which the measured data belongs to based on the comparing step is provided to the sensor controller 26 activating at step 46 the state indicator 19 to indicate the appropriate range for the vital sign signal value. Step 45 can be adopted, if desired, only in those cases the range is different from the previous state indication. If the state indication is same as previously indicated, it is not necessary to proceed from the comparing step 44 to step 45, but it is possible to return to step 40 to start by acquiring a new signal indicative of vital sign of the subject again. Thus at comparing step 44, it may be decided whether or not the vital sign signal value indicating the predetermined range covering the measured data is provided to the sensor controller 26. At step 46 the sensor controller activates the state indicator 19 indicating the appropriate range for the vital sign signal value received from the providing step 45.

Thus the range information may be provided to the sensor controller periodically, or constantly, for instance every 10 milliseconds; regardless of whether there has been a change from the current state. Or the range information may be compared to the previous state and if there has been a change a notification signal is provided to the sensor controller 26 activating the state indicator 19 to indicate a different range within which the vital sign signal value belongs to. The range information may be given together with the alarm provided that the vital sign signal value belongs to those data which should be alarmed or the range information may be in the form of the alarm only.

The written description uses examples to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. A sensor for physiological measurement, said sensor comprising:
at least one detector (21) for acquiring a signal indicative of vital sign of a subject;
**characterized in that** said sensor also comprising at least one state indicator (19) for indicating based on said signal acquired by said at least one detector one of at least two different ranges, which ranges are a first range and a second range, said first range being for one of desired vital sign signals and minor alarming vital sign signals, and said second range being for vital sign signals outside said first range.

2. The sensor according to claim 1, **characterized in that** said state indicator is one of visual indicator for displaying one of at least two different ranges and audio indicator for giving audible signal for at least one of at least two different ranges.

3. The sensor according to claim 2, **characterized in that** said visual indicator is a color indicator.

4. The sensor according to claim 1, **characterized in that** said first range is indicated without alarm and said second range is indicated with alarm.

5. The sensor according to claim 1, **characterized in that** said second range is for more alarming vital sign signals than said first range.

6. The sensor according to claim 1, **characterized in that** said second range is for medium priority vital sign signal alarm.

7. The sensor according to claim 6, **characterized in that** said at least one state indicator is configured to indicate a third range indicating vital sign signals which are still more alarming, such as high priority alarm, than the second range.

8. The sensor according to claim 1, further comprising a first signal connection (23) for transmitting said signal to a processing unit (31) for subsequent calculation of said vital sign.

9. The sensor according to claim 8, further comprising a second signal connection (24) for guiding said state indicator (19) based on calculation made by said processing unit to indicate the range of the vital sign signal.

10. The sensor according to claim 1 or 9, further comprising a sensor controller (26) to activate said state indicator (19).

11. The sensor according to claim 10, **characterized in that** said first and said second signal connections are one of wireless and wire line.

12. The sensor according to claim 1, **characterized in that** the signal frequency acquired by said at least one detector is more than 0,1 Hz.

13. A monitoring system for physiological measurement, said monitoring system comprising:
a sensor (20) having at least one detector (21) for acquiring a signal indicative of vital sign of a subject;
a monitor unit (30) having processing unit (31) with a measurement algorithm (32) to calculate said signal received from said at least one detector, and a user interface (34) for controlling said processing unit; and
a first signal connection (23) for transmitting said signal received by said at least one detector to said processing unit,
**characterized in that** said sensor also comprising at least one state indicator (19) for indicating one of at least two different ranges, which ranges are a first range and a second range, said first range being for one of desired vital sign signals and minor alarming vital sign signals, and said second range being for vital sign signals outside said first range, and that said monitoring system also comprising a second signal connection (24) for guiding said state indicator (19) based on calculation made by said processing unit to indicate the range of the vital sign signal.

14. The monitoring system according to claim 13, further comprising a sensor controller (26) to activate said state indicator (19).

15. A method for physiological measurement, said method comprising:
acquiring (40) by means of a sensor (20) a signal indicative of vital sign of a subject;
providing (41) said signal to a processing unit (31) provided with a measurement algorithm (32); and
calculating (42) by means of said measurement algorithm a vital sign signal value based on said signal;
**characterized in that** said method also comprising:
determining (43) in said processing unit a range within which the vital sign signal value belongs to, provided that there are at least two different predetermined ranges, which are a first range and a second range, said first range being for one of desired vital sign signals and minor alarming vital sign signals, and said second range being for vital sign signals outside said first range;
comparing (44) in said processing unit (31) the determined range for the vital sign signal value to said at least two different predetermined ranges that have been set for the subject;
providing (45) based on said comparing a signal indicating the predetermined range covering the vital sign signal value to a sensor controller (26); and
activating (46) by means of said sensor controller a state indicator (19) of said sensor to indicate the appropriate range for the vital sign signal value.
